# EUROPEAN PATENT SPECIFICATION

(11) **EP 2 923 633 B1**
(45) Date of publication and mention of the grant of the patent: **12.07.2017**
(21) Application number: 15159670.7
(22) Date of filing: 18.03.2015
(51) Int. Cl.: A61B 1/00, A61B 1/005

(54) **ENDOSCOPE**
ENDOSKOP
ENDOSCOPE

(30) Priority: 28.03.2014 JP 2014069810
(43) Date of publication of application: 30.09.2015
(73) Proprietor: Fujifilm Corporation, Minato-ku Tokyo 106-8620 (JP)
(72) Inventor: Ueda, Yoshihiro, Kanagawa, 258-8538 (JP)
(74) Representative: Klunker IP Patentanwälte PartG mbB

(56) References cited:
- JP-A- H11 262 470
- JP-A- 2002 360 504
- JP-A- 2003 000 533
- JP-A- 2011 120 687
- US-A1- 2002 032 368

## Description

### BACKGROUND OF THE INVENTION

### 1. Field of the Invention

The present invention relates to an endoscope in which the flexibility of a flexible portion varies.

### 2. Description of the Related Art

An endoscope includes an insertion unit which is inserted into the body of a subject, and a hand operation unit which is connected to the base end of the insertion unit. The insertion unit includes a tip portion, a bending portion, and a flexible portion arranged in order from the tip of the insertion unit. The bending portion has a structure in which a plurality of bending pieces are connected to one another, and is operated to be bent by pushing and pulling a bending operation wire which is provided in the bending pieces, thereby changing the direction of the tip portion.

The flexible portion has flexibility so as to be inserted into an insertion path which is bent with complexity. However, due to the flexibility, it is difficult to determine the direction or the posture of the tip portion, and thus it is difficult to introduce the tip portion toward a target site. Here, an endoscope is known in which a tightly wound coil spring as a hardness varying member is disposed in a flexible portion and the tightly wound coil spring is compressed by pulling a hardness varying wire which is inserted into the tightly wound coil spring such that the hardness thereof is changed (JP2003-000533A, JP2008-245925A, and JP2001-258828).

The tip of the tightly wound coil spring is fixed to the tip of the hardness varying wire and is attached to the tip of the flexible portion, and the base end of the tightly wound coil spring is fixed to a fixing member which is provided in the hand operation unit. In addition, the base end of the hardness varying wire is connected to a wire pulling unit which is disposed in the hand operation unit.

### SUMMARY OF THE INVENTION

The tip side and the base end side of the tightly wound coil spring which is disposed in the flexible portion are fixed, and thus when the flexible portion is in a bent state, portions in the vicinity of the center of the flexible portion receive forces from both end sides and try to move in the radial direction. The tightly wound coil spring which moves in the radial direction abuts and compresses other built-in components which are inserted into the flexible portion. Into the flexible portion, a plurality of built-in components having different outer diameters or hardnesses such as cables or treatment tool insertion pipes are inserted. Therefore, the built-in components which abut the tightly wound coil spring in a direction in which the flexible portion is bent vary, and thus anisotropy occurs in bending hardness. When anisotropy occurs in the bending hardness of the flexible portion, there may be a case where the flexible portion is less likely to be bent even when the same force is applied thereto or the flexible portion is bent too much, and thus an operator feels discomfort.

In view of the above, in the endoscopes of JP2003-000533A and JP2008-245925A, the tightly wound coil spring is attached to the tip of the flexible portion to be movable in the longitudinal direction (tube center direction) via a connecting member which is provided in the tip of the tightly wound coil spring. Accordingly, other built-in components or the flexible portion is prevented from being compressed by the tightly wound coil spring. In addition, in the endoscope of JP2001-258828, the tightly wound coil spring is attached to the tip of the flexible portion via a connecting coil which is provided in the tip of the tightly wound coil spring.

However, in the endoscopes described in JP2003-000533A, JP2008-245925A, and JP2001-258828, when the flexible portion is in a bent state, the tightly wound coil spring may catch on other built-in components at a position to which the tightly wound coil spring moves in the tube center direction and thus does not return to its original position. As described above, since the built-in components are different from each other in hardness and outer diameter, a case where the tightly wound coil spring does not return to its original position causes an increase in bending hardness and thus causes anisotropy. An endoscope according to the preamble of claim 1 is disclosed in JP2003-000533 A.

An object of the endoscope of the present invention is to provide an endoscope which suppresses anisotropy which occurs in the bending hardness of a flexible portion.

The above object is achieved by the endoscope according to claim 1. An endoscope according to an aspect of the present invention includes an insertion unit, a hardness varying member, a hardness varying wire, and a relay member. The insertion unit includes a tip portion, a bending portion, and a flexible portion arranged in order from a tip. The hardness varying member is disposed in the flexible portion and has a hardness that increases in accordance with compression in a tube center direction. The hardness varying wire is inserted into the hardness varying member and one end thereof is fixed to one end of the hardness varying member. The hardness varying wire compresses the hardness varying member by a pulling operation. The relay member is provided between one end of the hardness varying member and a connecting ring at a tip of the flexible portion or a bending piece. The relay member is more flexible than the hardness varying member, and is attached in a state of having deflection when the flexible portion is in a straight state. Alternatively, the relay member is more flexible than the hardness varying member, is made of an elastic material, and is attached in an extended state when the flexible portion is in a straight state.

In addition, the relay member may include a sliding portion and a rotation restricting portion. The sliding portion is provided in one end of the relay member is slidably attached to the connecting ring or the bending piece. The rotation restricting portion restricts rotation of the sliding portion with respect to the connecting ring or the bending piece.

The sliding portion may include a projection portion which protrudes in a radial direction of the hardness varying member. The rotation restricting portion may be constituted by the projection portion and a groove into which the projection portion is slidably fitted. The sliding portion may have a polygonal cross-section. The rotation restricting portion may be constituted by the sliding portion and a hole into which the sliding portion is slidably fitted and which has a polygonal cross-section. The sliding portion may have an elliptical cross-section. The rotation restricting portion may include the sliding portion and a hole into which the sliding portion is slidably fitted and which has an elliptical cross-section.

The sliding portion may be a cylindrical member which is provided in one end of the hardness varying member and into which a columnar member provided in the connecting ring or the bending piece is slidably fitted. The rotation restricting portion may be formed in an outer peripheral surface of the columnar member and an inner peripheral surface of the sliding portion.

The sliding portion may be slidably attached to a plurality of bending pieces. At least a portion of the relay member may include a plate spring or a coil spring.

The endoscope may further include an attachment portion to which the relay member is attached. The attachment member may be provided in one end of the hardness varying member and may be disposed at a center of the connecting ring or the bending piece in a radial direction.

In the endoscope according to the aspect of the present invention, since the hardness varying member is attached to the connecting ring or the bending piece via the relay member which has higher flexibility than that of the hardness varying member and is attached in a state of having deflection or in an extended state when the flexible portion is in a straight state, the hardness varying member returns to its original position when the flexible portion enters the straight state from the bent state. Furthermore, since the hardness varying member moves in the tube center direction when the flexible portion is in the bent state, the occurrence of anisotropy in the bending hardness of the flexible portion is suppressed.

### BRIEF DESCRIPTION OF THE DRAWINGS

Fig. 1 is a perspective view illustrating an embodiment of an endoscope system.
Fig. 2 is a perspective view illustrating a tip portion of an insertion unit.
Fig. 3 is a cross-sectional view of the main parts of a bending portion and a flexible portion.
Fig. 4 is a cross-sectional view of the flexible portion in a direction perpendicular to a tube center direction.
Fig. 5 is a perspective view illustrating the attachment structure of a plate spring.
Fig. 6 is a perspective view illustrating the configuration of a pulling unit.
Fig. 7A is an explanatory view illustrating the insertion unit when the flexible portion is in a straight state.
Fig. 7B is an explanatory view illustrating the insertion unit in a case where a hardness varying member is moved when the flexible portion is in a bent state.
Fig. 8 is a cross-sectional view of the main parts of a bending portion and a flexible portion of a second embodiment.
Fig. 9 is a perspective view illustrating the attachment structure of a connecting coil spring of the second embodiment.
Fig. 10 is a cross-sectional view of the main parts of a bending portion and a flexible portion of a third embodiment.
Fig. 11 is a cross-sectional view taken along line XI-XI of Fig. 10.
Fig. 12 is a cross-sectional view illustrating a first modification example of the third embodiment.
Fig. 13 is a cross-sectional view illustrating a second modification example of the third embodiment.
Fig. 14 is a cross-sectional view of the main parts of a bending portion and a flexible portion of a fourth embodiment.
Fig. 15 is a cross-sectional view of the main parts of a bending portion and a flexible portion of a fifth embodiment.
Fig. 16 is a cross-sectional view of the main parts of a bending portion and a flexible portion of a sixth embodiment.
Fig. 17 is a perspective view illustrating the attachment structure of a plate spring of the sixth embodiment.

### DESCRIPTION OF THE PREFERRED EMBODIMENTS

### First Embodiment which does not form part of the invention.

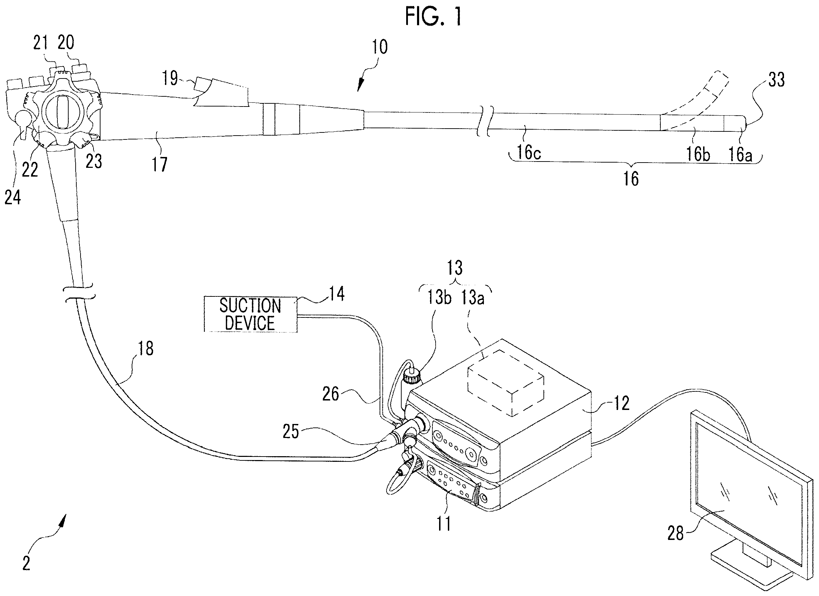

As illustrated in Fig. 1, an endoscope system 2 includes an electronic endoscope 10, a processor device 11, a light source device 12, an air/water supply device 13, and a suction device 14. The air/water supply device 13 includes a well-known air supply device (a pump or the like) 13a which is built in the light source device 12 and supplies gas, and a washing water tank 13b which is provided outside the light source device 12 and stores washing water. The electronic endoscope 10 includes an insertion unit 16 which is inserted into the body, a hand operation unit 17 which is connected to the base end portion of the insertion unit 16, and a universal cord 18 which is connected to the processor device 11 or the light source device 12.

The insertion unit 16 includes a tip portion 16a, a bending portion 16b, and a flexible portion 16c arranged in this order from the tip. A camera unit (not illustrated) for photographing the inside of the body of a subject is built in the tip portion 16a. The bending portion 16b is connected to the base end of the tip portion 16a, and is configured to be bendable. The flexible portion 16c is connected to the base end of the bending portion 16b, and has flexibility.

In the hand operation unit 17, a treatment tool inlet port 19, an air/water supply button 20, a suction button 21, bending operation knobs 22 and 23 as bending operation members, and an operating lever 24 are provided. A connector 25 is attached to the other end of the universal cord 18. The connector 25 is a complex type connector, and is connected to each of the processor device 11, the light source device 12, and the air/water supply device 13. The suction device 14 is connected to the connector 25 via a connecting tube 26.

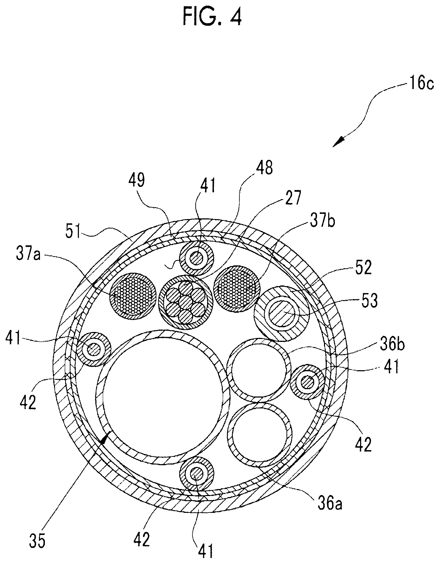

The processor device 11 is electrically connected to the light source device 12, and collectively controls the operations of the endoscope system 2. The processor device 11 supplies power to the electronic endoscope 10 via a signal cable 27 (see Fig. 4) which is inserted into the universal cord 18 and the insertion unit 16, and controls the driving of the camera unit. In addition, the processor device 11 acquires an imaging signal which is output from the camera unit via the signal cable, and generates image data by performing various image processes thereon. The image data generated by the processor device 11 is displayed on a monitor 28 which is connected to the processor device 11 via a cable as an observation image.

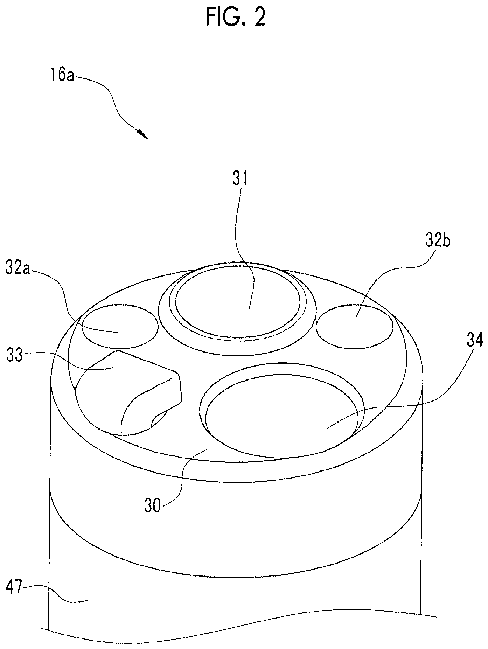

As illustrated in Fig. 2, a tip surface 30 of the tip portion 16a is provided with an observation window 31, illumination windows 32a and 32b, an air/water supply nozzle 33, and a treatment tool outlet port 34. The observation window 31 is also used as a cover glass, and is an objective lens at the outermost tip of the camera unit.

In the insertion unit 16, an air/water supply conduit (not illustrated) and a treatment tool insertion conduit 35 (see Fig. 4) are arranged. One end of the air/water supply conduit communicates with the air/water supply nozzle 33. The other end of the air/water supply conduit branches off into an air supply conduit 36a (see Fig. 4) and a water supply conduit 36b (see Fig. 4). The air supply device 13a supplies gas (air or carbon dioxide gas) during endoscopy using the electronic endoscope 10. When an air supply operation is performed by the air/water supply button 20, gas generated by the air supply device 13a is sent to the air supply conduit 36a. When a water supply operation is performed, washing water is sent from the washing water tank 13b to the water supply conduit 36b by the pressure of the gas generated by the air supply device 13a. The air/water supply nozzle 33 selectively ejects the gas and the washing water which are supplied via the air supply conduit 36a, the water supply conduit 36b, and the air/water supply conduit.

One end of the treatment tool insertion conduit 35 communicates with the treatment tool outlet port 34, and the other end thereof is connected to the treatment tool inlet port 19. The treatment tool inlet port 19 allows various treatment tools in which an injection needle, a high-frequency scalpel, or the like is provided at the tip thereof to be inserted thereinto, and is blocked by a stopper (not illustrated) unless a treatment tool is inserted therein. When a suction operation is performed by the suction button 21, suction from the treatment tool outlet port 34 is performed due to negative pressure generated by the suction device 14. In addition, when a blocking operation is performed, the negative pressure is shut off, and thus the suction from the treatment tool outlet port 34 is stopped.

The illumination windows 32a and 32b are also used as irradiation lens, and allow illumination light from the light source device 12 to illuminate an observation site inside the body. The illumination windows 32a and 32b respectively face the emission ends of light guides 37a and 37b (see Fig. 4). The light guides 37a and 37b are formed by binding a large number of optical fibers together. The light guides 37a and 37b guide the illumination light from the light source device 12 to the illumination windows 32a and 32b through the insides of the insertion unit 16, the hand operation unit 17, the universal cord 18, and the connector 25. In addition, as the light which is guided from the light source device 12, for example, excitation light such as laser light may be used. In this case, a type in which the excitation light from the light source device 12 is guided by a single optical fiber and causes a fluorescent body disposed in the tip portion 16a to emit illumination light is preferable.

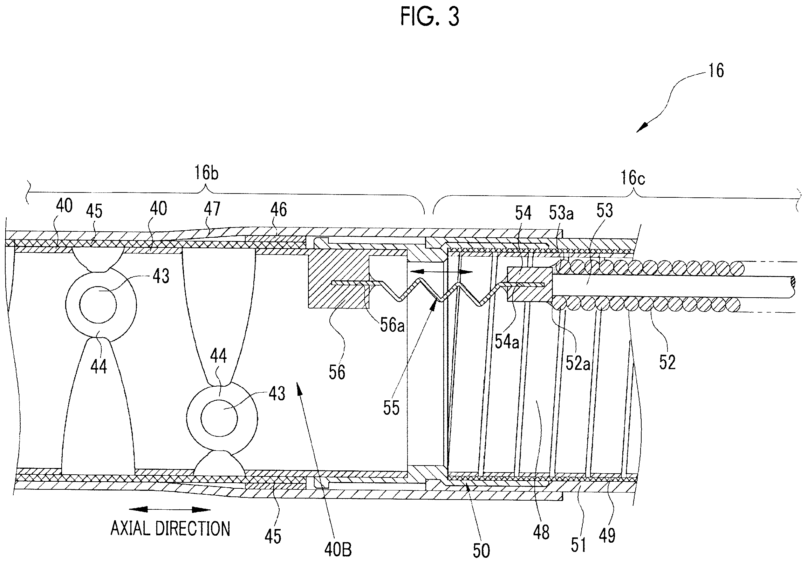

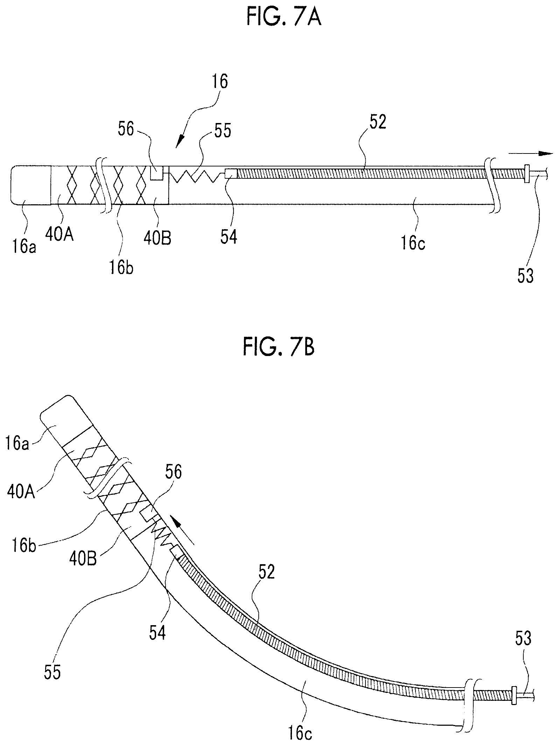

As illustrated in Fig. 3, the bending portion 16b includes a plurality of (for example, 16) bending pieces 40 which are connected in series. Hereinafter, for the convenience of description, among the plurality of bending pieces 40, the bending piece which is positioned on the outermost tip side of the bending portion 16b is referred to as a tip piece 40A (see Figs. 7A and 7B), and the bending piece which is positioned on the outermost base end side of the bending portion 16b is referred to as a base end piece 40B. The tip piece 40A is fixed to the tip portion 16a, and the base end piece 40B is fixed to the flexible portion 16c. Bending operation wires 41 (see Fig. 4) which extend from the hand operation unit 17 to the bending portion 16b through the flexible portion 16c are connected to the bending pieces 40. In addition, in Fig. 3, in order to prevent complexity of the figure, illustration of the treatment tool insertion conduit 35, the air supply conduit 36a, the water supply conduit 36b, the light guides 37a and 37b, the bending operation wires 41, a guide pipe 42, and the like is omitted.

The bending piece 40 is made of metal, and the plurality of bending pieces 40 are connected by connecting pins 43 which are the rotation centers of the bending pieces 40. The base end piece 40B is connected to a connecting ring 50 of the flexible portion 16c. Each of the bending pieces 40 except for the tip piece 40A and the base end piece 40B is provided with a pair of tongue pieces 44 into which the connecting pin 43 is inserted, and which are provided on both the tip side and the rear end side. In a case where the tongue pieces 44 are vertically provided on the tip side, the tongue pieces 44 are horizontally provided on the base end side such that the tongue pieces 44 are provided vertically and horizontally in an alternate manner. The vertical or horizontal tongue pieces 44 of the adjacent bending pieces 40 are connected to each other by the connecting pin 43.

The outer periphery of the bending piece 40 is covered with a braid 45. The outer peripheral surface of the end portion of the braid 45A is fitted into a metal ring 46. In addition, in Fig. 3, the metal ring 46 is provided only on the base end portion of the braid 45, but may be provided only on the tip portion of the braid 45. The outer periphery of the braid 45 is covered with a covering tube 47, which is an outer covering formed of rubber in a cylindrical shape. The braid 45 is a mesh-like body which is formed by braiding together a plurality of wires made of metal. The braid 45 covers the plurality of bending pieces 40 which are connected, and thus stabilizes the posture of each of the bending pieces 40. In addition, the inner peripheral surface of the covering tube 47 comes into contact with the braid 45, and thus the adhesion therebetween is enhanced.

The tip side of the covering tube 47 also covers the base end side of the tip portion 16a. The tip of the covering tube 47 is fixed to the tip portion 16a by winding, for example, thread (not illustrated) around the tip. A sealing material or an adhesive may be applied to the point around which thread is wound, and is hardened. In addition, the base end side of the covering tube 47 also covers a range to the tip side of the flexible portion 16c.

As illustrated in Fig. 4, the two groups of bending operation wires 41 for the vertical arrangement and the horizontal arrangement are provided, and the base end sides of the bending operation wires 41 are wound around pulleys which are rotated in connection with the operations of a vertical bending operation knob 22 and a horizontal bending operation knob 23 that are provided in the hand operation unit 17. In addition, in the flexible portion 16c, guide pipes 42 as guide members which guide the bending operation wires 41 are provided.

When the vertical bending operation knob 22 is operated to rotate, the bending operation wires 41 for the vertical arrangement are pushed and pulled, and thus the bending portion 16b is operated to bend in the vertical direction. When the horizontal bending operation knob 23 is operated to rotate, the bending operation wires 41 are pushed and pulled, and thus the bending portion 16b is operated to bend in the horizontal direction. Accordingly, the tip portion 16a can be directed in a desired direction in the body.

The flexible portion 16c includes a spiral pipe 48 which is formed by winding a band plate made of metal such as stainless steel in a spiral shape, a braid 49 which covers the outer periphery of the spiral pipe 48 and is a mesh-like body formed by braiding together wires made of metal such as stainless steel in a net shape, the connecting rings 50 (see Fig. 3) which are made of metal such as stainless steel and are fixed to the outer peripheral surfaces of both end portions of the braid 49, and an outer covering 51 which is made of a resin and is extruded to cover the outer periphery of the braid 49. The connecting rings 50 are positioned on the outermost tip side and base end side of the flexible portion 16c. The connecting ring 50 on the tip side is connected to the bending portion 16b, and the connecting ring 50 on the base end side is connected to the hand operation unit 17. The outer periphery of the braid 49 is fitted into the connecting ring 50, and a portion of the connecting ring 50 and the braid 49 are covered with the outer covering 51. In addition, in the configuration of the flexible portion 16c, the outer covering 51 may directly cover the outer peripheral surface of the spiral pipe 48 without the braid 49.

The outer peripheral surface of the base end piece 40B is fitted into the inner peripheral surface of the connecting ring 50 on the tip side. In the assembly process of connecting the bending portion 16b and the flexible portion 16c to each other, the outer peripheral surface of the base end piece 40B is fitted into the inner peripheral surface of the connecting ring 50, and the base end piece 40B and the connecting ring 50 are joined to each other through soldering or the like. In addition, the method of joining the base end piece 40B and the connecting ring 50 to each other is not limited to soldering, and may be a joining method such as screwing together or engaging metal components.

In the flexible portion 16c, a tightly wound coil spring 52 as a hardness varying member and a hardness varying wire (hereinafter, referred to as a wire) 53 which is inserted into the tightly wound coil spring 52 are arranged. The tightly wound coil spring 52 is a coil spring in which a metal wire is closely wound, and the hardness thereof varies with compression in the tube center direction. The wire 53 applies a compressive force to the tightly wound coil spring 52 through a pulling operation of a wire pulling unit 60, which will be described later, such that the hardness of the tightly wound coil spring 52 is increased. The tightly wound coil spring 52 is positioned in the vicinity of the inner peripheral surface of the flexible portion 16c and is disposed along the inner peripheral surface of the spiral pipe 48.

In addition, in the flexible portion 16c, in addition to the tightly wound coil spring 52 and the hardness varying wire 53, the signal cable 27, the light guides 37a and 37b, the treatment tool insertion conduit 35, the bending operation wires 41, the guide pipes 42, the air supply conduit 36a, and the water supply conduit 36b are provided as built-in components.

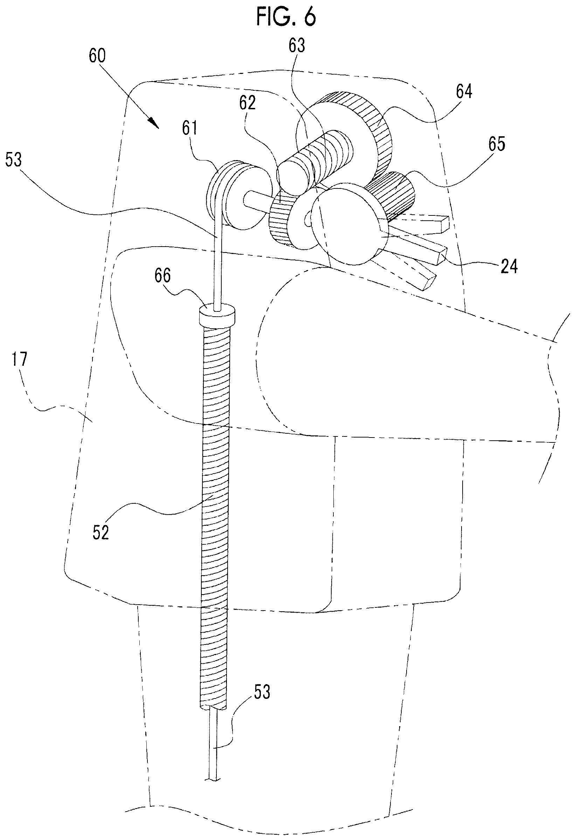

As illustrated in Fig. 3, a tip portion 53a of the wire 53 which is inserted into the tightly wound coil spring 52 is fixed to a tip portion 52a of the tightly wound coil spring 52, and a base end portion thereof is connected to the wire pulling unit 60 (see Fig. 6). The tip portion 53a of the wire 53 and the tip portion 52a of the tightly wound coil spring 52 are strongly fixed to each other through brazing. In addition, the fixing method is not limited thereto and may be performed through adhesion using an adhesive or the like. In addition, when the operating lever 24 which is provided at the base end portion of the hand operation unit 17 is operated to be pulled, the wire 53 is pulled by the wire pulling unit 60. As a result, the tightly wound coil spring 52 is compressed, and thus the tightly wound coil spring 52 is changed to be in a high hardness state. Accordingly, the flexible portion 16c is adjusted to be in a state where the flexible portion 16c has low flexibility and is less likely to be bendable. In addition, the tip of the tightly wound coil spring 52 is attached at a position of 15 cm or more and 30 cm or less from the tip of the tip portion 16a when the flexible portion 16c is in a straight state.

The tip portions 52a and 53a of the tightly wound coil spring 52 and the wire 53 are attached to the base end piece 40B via a connecting member 54 and a plate spring 55 as a relay member. The connecting member 54 is integrally fixed to the tips of the wire 53 and the tightly wound coil spring 52 through brazing. The base end piece 40B is integrally provided with an attachment portion 56 which protrudes from the inner peripheral surface thereof

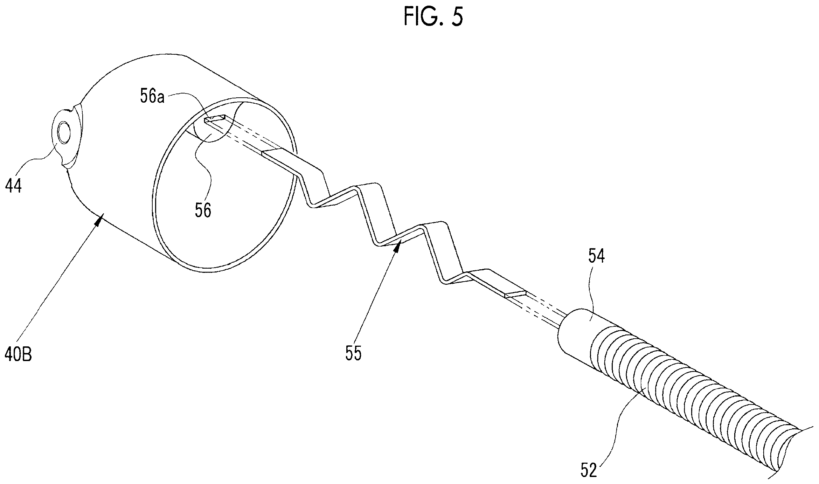

As illustrated in Fig. 5, the plate spring 55 is formed by folding a metal plate, and is more flexible than the tightly wound coil spring 52. Opening portions 54a and 56a (see Fig. 3) which match the width and the thickness of the plate spring 55 are respectively formed in the tip of the connecting member 54 and the base end of the attachment portion 56. The base end portion and the tip portion of the plate spring 55 are fitted into the opening portions 54a and 56a and are fixed to the connecting member 54 and the attachment portion 56 through brazing, soldering, or the like. Accordingly, when the flexible portion 16c is in a straight state, the plate spring 55 is attached in a state of having deflection. In the plate spring 55 attached in the state of having deflection, an elastic force is generated when the tightly wound coil spring 52 moves toward the tip side. In addition, "state of having deflection" mentioned above indicates a state of having a bent portion in the tube center direction of the tightly wound coil spring 52, and for example, may be formed with a plurality of folded portions as in the plate spring 55 or may be formed with a single folded portion.

When the flexible portion 16c is in a bent state, the tightly wound coil spring 52 of which the tip portion 52a is attached via the plate spring 55 moves toward the tip side in the tube center direction against the elastic force of the plate spring 55. On the other hand, when the flexible portion 16c returns to the straight state from the bent state, the tightly wound coil spring 52 moves toward the base end side in the tube center direction due to the elastic force of the plate spring 55 and returns to its original position In addition, the size of the plate spring 55 in the tube center direction is formed to be short, and for example, the length of the plate spring 55 is formed to be 5 cm or more and 20 cm or less while the length of the tightly wound coil spring 52 is 100 cm or more and 150 cm or less.

As illustrated in Fig. 6, the wire pulling unit 60 for pulling the wire 53 which is inserted into the tightly wound coil spring 52 is provided in the hand operation unit 17. The wire pulling unit 60 includes a wire winding pulley (hereinafter, simply referred to as a pulley) 61, a worm wheel 62, a worm 63, a spur gear 64, and a gear 65. The wire 53 is wound around the pulley 61. In addition, the pulley 61 is coaxially connected to the worm wheel 62.

The worm wheel 62 meshes with the worm 63. The spur gear 64 is coaxially connected to the worm 63, and the spur gear 64 meshes with the gear 65 which is joined to the operating lever 24. The operating lever 24 is attached to the hand operation unit 17 so as to be rotated. In addition, the operation unit for varying hardness is not limited to the operating lever 24 for a rotating operation, and an operation unit of a knob type, a dial type, or the like may also be used.

The base end portion of the wire 53 is fixed to the pulley 61. In addition, a fixing member 66 to which the tightly wound coil spring 52 is fixed is provided in the vicinity of the pulley 61 which is disposed at the base end portion of the hand operation unit 17. The fixing member 66 to which the tightly wound coil spring 52 is provided in the hand operation unit 17, and thus the tightly wound coil spring 52 extends to the inside of the hand operation unit 17.

When the operating lever 24 is operated by an operator, the gear 65 which is joined to the operating lever 24 is driven, and correspondingly the spur gear 64 is driven. As a result, the worm 63 which is coaxially joined to the spur gear 64 is driven. In addition, the worm wheel 62 is driven by the worm 63, the pulley 61 is rotated, and the wire 53 is pulled.

In addition, since the tip of the wire 53 is fixed to the tip of the tightly wound coil spring 52 and the base end of the tightly wound coil spring 52 is fixed to the fixing member 66, when the wire 53 is pulled, the tightly wound coil spring 52 is pulled toward the pulley 61 side of the wire pulling unit and is compressed between the tip thereof and the fixing member 66, and thus the hardness thereof is increased.

In addition, the operating lever 24 is configured to be operated in the upward direction and the downward direction as illustrated by two-dot chain lines. When the operating lever 24 is operated in the upward direction, the spur gear 64 is driven by the gear 65, the worm 63 is driven along with the spur gear 64, the worm wheel 62 is driven by the worm 63, the pulley 61 is rotated in a direction in which the wire 53 is wound, the tightly wound coil spring 52 is compressed as the wire 53 is pulled, the hardness of the tightly wound coil spring 52 is increased, and the hardness of the flexible portion 16c is increased (flexibility is decreased). In addition, when the operating lever 24 is operated in the downward direction, each of the gears is driven in the reverse direction to the above-described direction, the pulley 61 is rotated in a direction in which the wire 53 is unwound, the compression of the tightly wound coil spring 52 is released as the wire 53 is relaxed, the hardness of the tightly wound coil spring 52 is decreased, and the hardness of the flexible portion 16c is also decreased (flexibility is increased).

The action of the above configuration will be described. After preparation for inspection using the endoscope system 2 is completed, the insertion unit 16 is inserted into the body, for example, into the digestive tract. When it becomes difficult to insert the insertion unit 16 into a deep portion of the tract in the body, the operating lever 24 is rotated. In this case, as illustrated in Fig. 7A, the hardness varying wire 53 is pulled, the hardness of the tightly wound coil spring 52 is increased, and the hardness of the flexible portion 16c is increased. Accordingly, the force of the operator is easily transmitted to the flexible portion 16c, and thus the insertion unit 16 can be pushed to advance toward the deep portion of the tract. Furthermore, when the insertion unit 16 is inserted into the deep portion of the tract, in order to introduce the tip portion 16a toward a target site, the flexible portion 16c needs to be bent in various directions.

As illustrated in Fig. 7B, when the flexible portion 16c is in the bent state, since the tip portions 52a and 53a of the tightly wound coil spring 52 and the wire 53 are attached to the base end piece 40B via the plate spring 55, the tightly wound coil spring 52 which is bent along with the flexible portion 16c moves toward the tip side in the tube center direction against the elastic force of the plate spring 55. Accordingly, the movement of the tightly wound coil spring 52 in the radial direction is restricted, and thus the tightly wound coil spring 52 does not compress other built-in components, thereby suppressing anisotropy which occurs in the bending hardness of the flexible portion 16c. Furthermore, when the flexible portion 16c returns to the straight state from the bent state, the tightly wound coil spring 52 returns to its original position due to the elastic force of the plate spring 55, and thus the anisotropy which occurs in the bending hardness of the flexible portion 16c can be further suppressed. In addition, since the size of the plate spring 55 in the tube center direction of the tightly wound coil spring 52 is formed to be short, the plate spring 55 is not twisted even when the flexible portion 16c is bent in various directions. There may be a case where, when the tightly wound coil spring 52 is loosened, the hardness thereof is reduced. However, as described above, since the rotation of the tightly wound coil spring 52 in the axial direction thereof is restricted, the tightly wound coil spring 52 can be prevented from being loosened.

### Second Embodiment which does not form part of the invention.

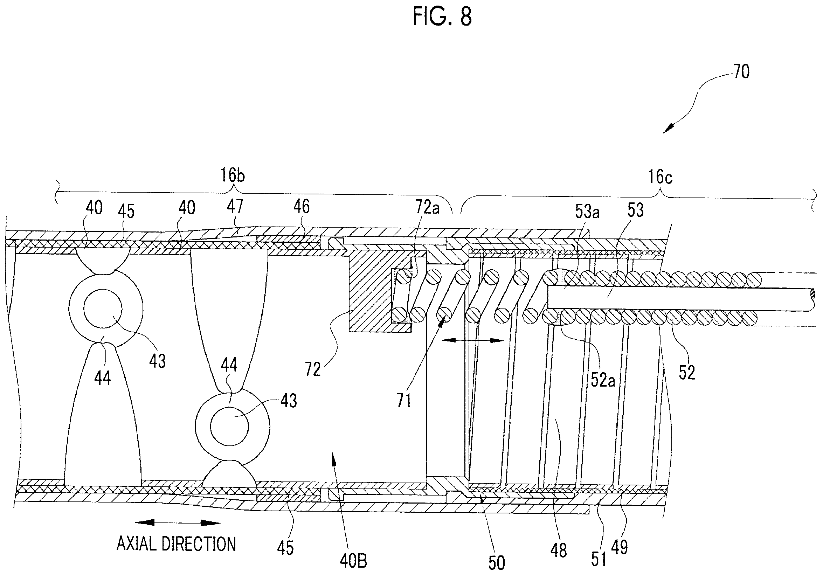

In the endoscope of the first embodiment, the example in which the tip portion of the tightly wound coil spring 52 is attached via the plate spring 55 as a relay member is described. However, the present invention is not limited thereto, and a configuration in which a connecting coil spring 71 is used as the relay member may be employed as in an insertion unit 70 of a second embodiment illustrated in Fig. 8. In this case, the connecting coil spring 71 is elastically formed by winding a metal wire and has higher flexibility than that of the tightly wound coil spring 52. The base end portion of the connecting coil spring 71 is integrally fixed to the tips of the wire 53 and the tightly wound coil spring 52 through brazing. In addition, in a state where the tip portion 53a of the wire 53 is fitted into the inner peripheral surface of the connecting coil spring 71, the base end portion of the connecting coil spring 71 may be fixed to the tips of the wire 53 and the tightly wound coil spring 52.

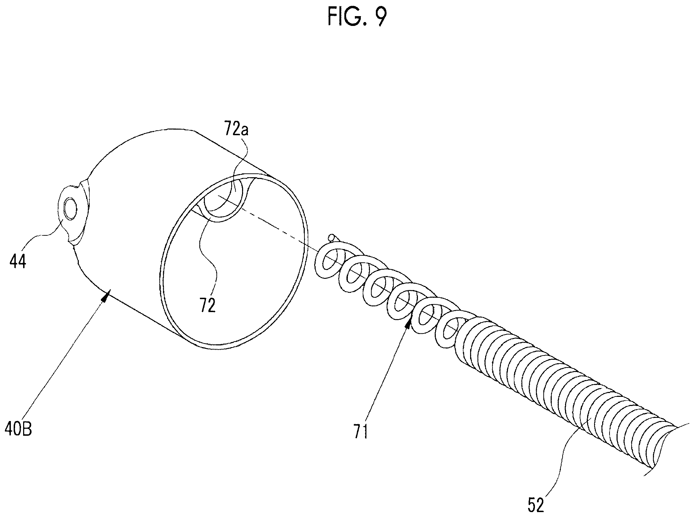

As illustrated in Fig. 9, the base end piece 40B is integrally provided with an attachment portion 72 which protrudes from the inner peripheral surface thereof. An opening portion 72a which matches the outer diameter of the connecting coil spring 71 is formed in the base end of the attachment portion 72. The tip portion of the connecting coil spring 71 is fitted into the opening portion 72a and is fixed to the attachment portion 72 through brazing, soldering, or the like. Accordingly, when the flexible portion 16c is in the straight state, the connecting coil spring 71 is attached in an extended state. In the connecting coil spring 71 which is attached in the extended state, an elastic force is generated when the tightly wound coil spring 52 moves toward the tip side. In addition, "extended state" mentioned above indicates a state where the metal wire which forms the connecting coil spring 71 is not closely wound and has gaps therein. In addition, as in the plate spring 55 of the first embodiment, the size of the connecting coil spring 71 in the tube center direction is formed to be shorter than that of the tightly wound coil spring 52.

When the flexible portion 16c is in the bent state, the tightly wound coil spring 52 of which the tip portion 52a is attached via the connecting coil spring 71 moves toward the tip side in the tube center direction against the elastic force of the connecting coil spring 71. On the other hand, when the flexible portion 16c returns to the straight state from the bent state, the tightly wound coil spring 52 moves toward the base end side in the tube center direction due to the elastic force of the connecting coil spring 71 and returns to its original position. Accordingly, as in the first embodiment, anisotropy which occurs in the bending hardness of the flexible portion 16c can be suppressed. In addition, in the second embodiment, the example in which the connecting coil spring 71 is used as the relay member is described. However, the present invention is not limited thereto, and a material which is elastic or is more flexible than the tightly wound coil spring 52 may be used. For example, rubber or the like may be used.

### Third Embodiment

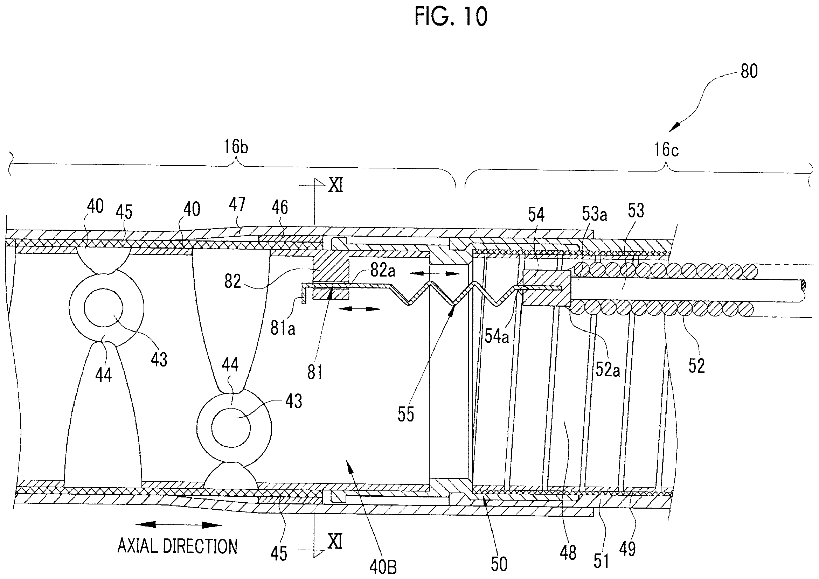

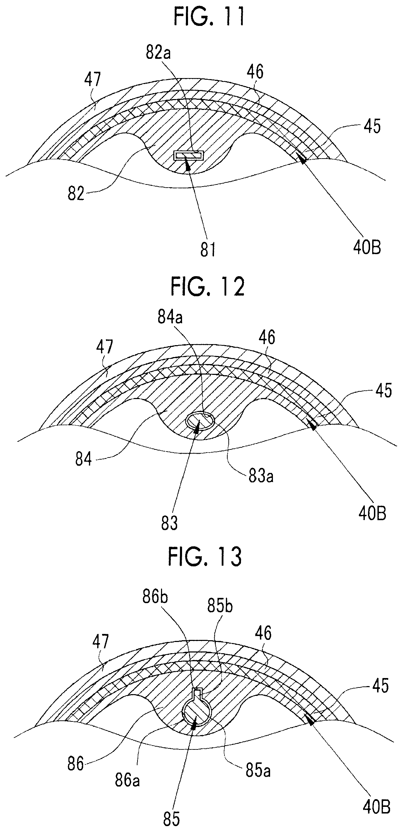

In the first and second embodiments, the example in which the tip portion of the plate spring 55 or the connecting coil spring 71 as the relay member is fixed to the base end piece 40B is described. However, the present invention is not limited thereto, and as in an insertion unit 80 of a third embodiment illustrated in Fig. 10, the tip of the relay member may be slidably attached to the base end piece 40B while the rotation thereof is restricted. In this case, a sliding portion 81 having a plate shape is formed integrally with the tip of the plate spring 55. The sliding portion 81 is formed of a metal plate like the plate spring 55. The base end piece 40B is integrally provided with an attachment portion 82 which protrudes from the inner peripheral surface thereof. As illustrated in Fig. 11, a through-hole 82a into which the sliding portion 81 is slidably fitted is formed in the attachment portion 82. The sliding portion 81 moves in the tube center direction of the tightly wound coil spring 52 along the through-hole 82a. A separation prevention portion 81a is formed in the sliding portion 81 at a position on the tip side which is fitted into the through-hole 82a and protrudes from the attachment portion 82. The separation prevention portion 81a is bent to be perpendicular to the sliding portion 81 and abuts the tip of the attachment portion 82 when the sliding portion 81 moves toward the base end side, thereby restricting the separation of the sliding portion 81.

In addition, the sliding portion 81 and the through-hole 82a are also used as rotation restricting portions. The through-hole 82a is formed to have a rectangular cross-section that matches the sliding portion 81. Accordingly, the rotation of the sliding portion 81 with respect to the base end piece 40B is restricted. The rotation of the tightly wound coil spring 52 about the axis is restricted by the rotation restricting portions and the plate spring 55.

As described above, since the tip of the plate spring 55 is slidably attached to the base end piece 40B via the sliding portion 81 while the rotation thereof is restricted, when the tightly wound coil spring 52 is bent along with the flexible portion 16c, the tightly wound coil spring 52 moves toward the tip side in the tube center direction against the elastic force of the plate spring 55, and furthermore, the plate spring 55 moves toward the tip side in the tube center direction of the tightly wound coil spring 52. Accordingly, as in the first and second embodiments, anisotropy which occurs in the bending hardness of the flexible portion 16c can be suppressed. Furthermore, a movement stroke generated when the tightly wound coil spring 52 is bent can be longer than those described in the first and second embodiments.

In the third embodiment, the example in which the sliding portion 81 is provided integrally with the plate spring 55 is described. However, the present invention is not limited thereto, and a configuration in which the sliding portion 81 is provided integrally with the connecting coil spring 71 as exemplified in the second embodiment and is slidably attached to the base end piece 40B while the rotation thereof is restricted may be employed.

In addition, in the third embodiment, the cross-sections of the sliding portion 81 and the through-hole 82a are formed in a rectangular shape. However, the present invention is not limited thereto, and a configuration in which the sliding portion and the attachment portion are slidably fitted to each other and are also used as rotation restricting portions may be employed. In addition, as in a sliding portion 83 and an attachment portion 84 illustrated in Fig. 12, an outer peripheral surface 83a of the sliding portion 83 and a through-hole 84a of the attachment portion 84 may have an elliptical shape, or may also have a polygonal shape other than the rectangular shape (quadrangular shape) such as a triangular shape or a pentagonal shape. Otherwise, as in a sliding portion 85 and an attachment portion 86 illustrated in Fig. 13, the sliding portion 85 may include a cylindrical portion 85a and a projection portion 85b which protrudes from the outer peripheral surface of the cylindrical portion 85a, and the attachment portion 86 may include a through-hole 86a into which the cylindrical portion 85a is fitted and a groove 86b into which the projection portion 85b is fitted such that the sliding portion 85 is slidable and the rotation thereof is restricted.

### Fourth Embodiment

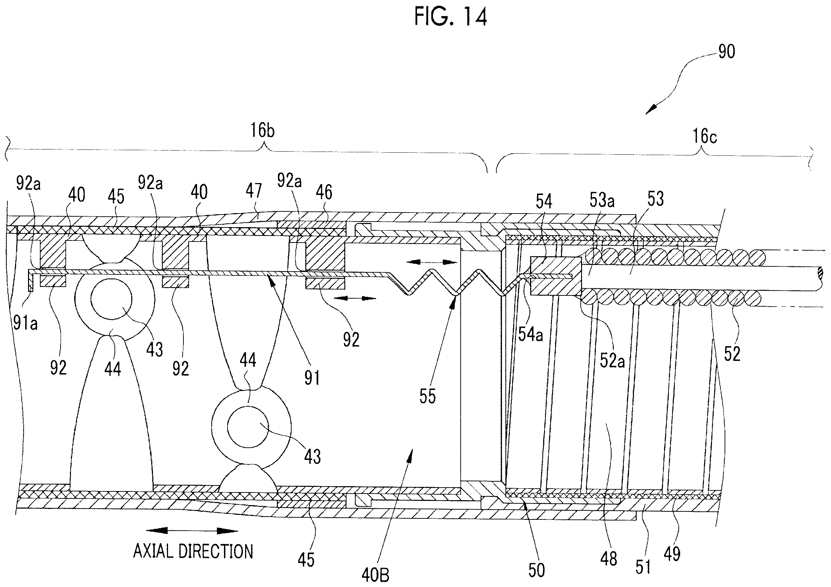

In the third embodiment, the example in which the sliding portion 81 is slidably attached to the base end piece 40B while the rotation thereof is restricted is described. However, the present invention is not limited thereto, and as in an insertion unit 90 of a fourth embodiment illustrated in Fig. 14, attachment portions 92 may be provided in the plurality of bending pieces 40 including the base end piece 40B, and a sliding portion 91 may be slidably attached to the attachment portions 92 while the rotation thereof is restricted. In this case, the sliding portion 91 having a plate shape is formed integrally with the tip of the plate spring 55. The sliding portion 91 is formed of a metal plate, and the size thereof in the longitudinal direction is formed to be long in order to be fitted into a plurality of attachment portions 92. A through-hole 92a into which the sliding portion 91 is slidably fitted is formed in each of the attachment portions 92. Accordingly, even in a case where the size of the base end piece 40B in the tube center direction is small, the sliding portion 91 can be attached, and the sliding portion 91 can be allowed to stably slide. A separation prevention portion 91a is formed in the sliding portion 91 at a position on the tip side which is fitted into the through-hole 92a and protrudes from the attachment portion 92 positioned at the outermost tip. The separation prevention portion 91a is bent to be perpendicular to the sliding portion 91 and abuts the tip of the attachment portion 92 when the sliding portion 91 moves toward the base end side, thereby restricting the separation of the sliding portion 91. In addition, as in the third embodiment, the sliding portion 91 and the through-holes 92a are also used as rotation restricting portions.

### Fifth embodiment

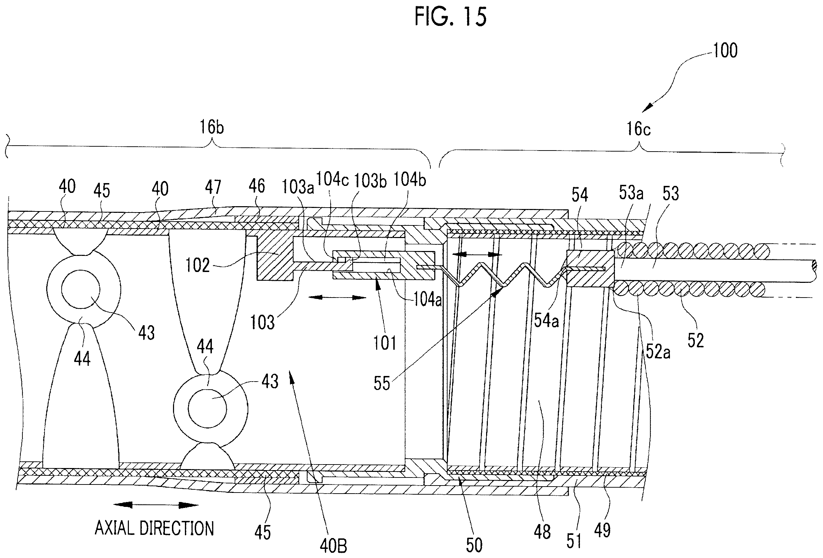

In the third and fourth embodiments, the sliding portion having a plate shape or a columnar shape is provided in the plate spring 55 and the connecting coil spring 71 as the relay members, and the through-hole is formed in the attachment portion provided in the bending piece 40 at least including the base end piece 40B so that the sliding portion is attached to the through-hole. However, the present invention is not limited thereto, and as in an insertion unit 100 of a fifth embodiment illustrated in Fig. 15, the sliding portion which is provided in one end of the relay member may be provided as a cylindrical member, and may be slidably attached to a columnar member provided in the bending piece 40 while the rotation thereof is restricted. In this case, a sliding portion 101 is provided integrally with the tip of the plate spring 55. The sliding portion 101 is a cylindrical member in which the base end side is blocked and the tip side is open. The sliding portion 101 is made of metal or a resin, and is fixed to the tip portion of the plate spring 55. The base end piece 40B is integrally provided with an attachment portion 102 which protrudes from the inner peripheral surface thereof. The attachment portion 102A is provided with a columnar member 103 which protrudes toward the base end side. The columnar member 103 is made of metal or a resin, and includes a cylindrical portion 103a and a projection portion 103b which protrudes from the outer peripheral surface of the cylindrical portion 103a. The sliding portion 101 includes an opening portion 104a and a groove 104b which is formed by cutting out the opening portion 104a. The opening portion 104a is formed along the axial direction from the tip of the sliding portion 101 so that the cylindrical portion 103a is slidably fitted thereinto. The projection portion 103b and the groove 104b are slidably fitted to each other, and are also used as rotation restricting portions. The rotation of the tightly wound coil spring 52 about the axis is restricted by the rotation restricting portions and the plate spring 55. In addition, a separation prevention portion 104c is formed on the tip side of the groove 104b. When the sliding portion 101 moves toward the base end side, the separation prevention portion 104c abuts the tip of the projection portion 103b and thus restricts the separation of the sliding portion 101.

In addition, in the fifth embodiment, the sliding portion 101 is slidably attached to the columnar member 103 which includes the cylindrical portion 103a and the projection portion 103b while the rotation thereof is restricted. However, the present invention is not limited thereto, and the columnar member and the opening portion of the sliding portion may have a polygonal shape or an elliptical shape.

Sixth Embodiment which does not form part of the invention.

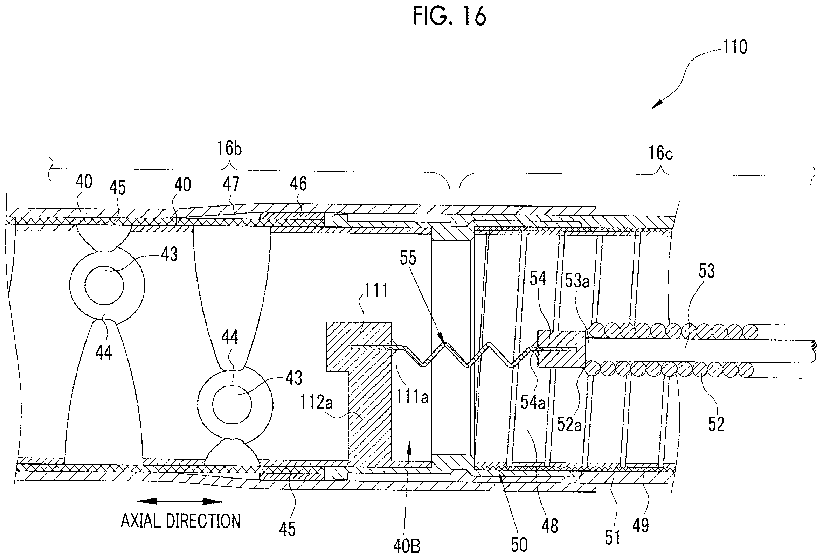

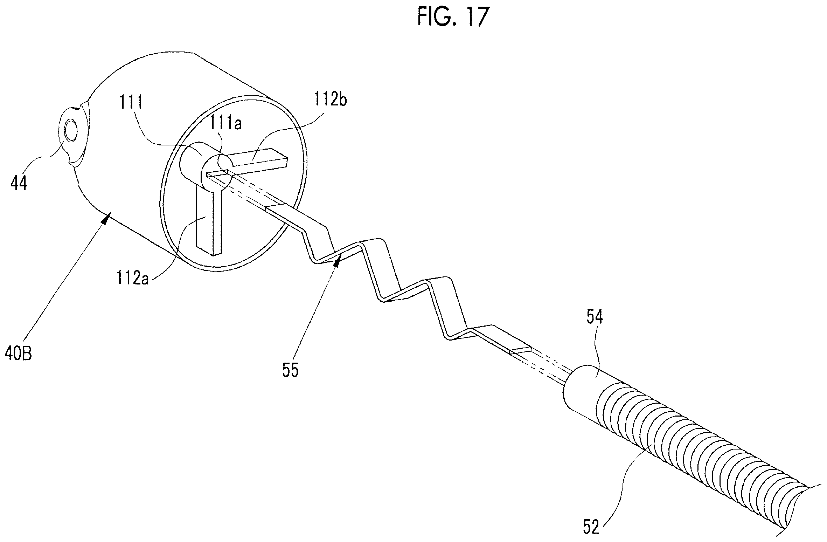

In the first to fifth embodiments, the example in which the tightly wound coil spring 52 as the hardness varying member is disposed in the vicinity of the inner peripheral surface of the flexible portion 16c is described. However, the present invention is not limited thereto, and as in an insertion unit 110 of a sixth embodiment illustrated in Fig. 16, the relay member may be attached to the center of the bending piece 40 in the radial direction. In this case, as illustrated in Fig. 17, an attachment portion 111 and two supporting posts 112a and 112b are provided integrally with the base end piece 40B. The attachment portion 111 is disposed at the center of the base end piece 40B in the radial direction, and the supporting posts 112a and 112b are positioned at an angle of 90° and protrude toward the outer peripheral surface of the attachment portion 111 from the inner peripheral surface of the base end piece 40B so as to connect the attachment portion 111 and the base end piece 40B to each other. In addition, the supporting posts which are disposed between the attachment portion 111 and the base end piece 40B are not limited thereto and may employ an arrangement in which there is a gap in the base end piece 40B to allow built-in components such as the tightly wound coil spring 52, the hardness varying wire 53, the signal cable 27, the light guides 37a and 37b, the treatment tool insertion conduit 35, the bending operation wires 41, the guide pipes 42, the air supply conduit 36a, and the water supply conduit 36b to be built therein. For example, three or four supporting posts may be arranged between the attachment portion 111 and the base end piece 40B at equal angles.

An opening portion 111a which matches the width and the thickness of the plate spring 55 is formed in the base end of the attachment portion 111. The tip portion of the plate spring 55 is fitted into the opening portion 111a and is fixed to the attachment portion 111 through brazing, soldering, or the like. In addition, the base end portion of the plate spring 55 is fixed to the connecting member 54 as in the first embodiment. Accordingly, the plate spring 55 is attached to the center of the flexible portion 16c in the radial direction, and as in the first embodiment, is attached in a state of having deflection when the flexible portion 16c is in the straight state. In addition, "the center of the flexible portion 16c in the radial direction" mentioned above includes a position substantially at the center thereof.

In the sixth embodiment, since the tightly wound coil spring 52 and the wire 53 are attached to the center in the radial direction of the flexible portion 16c, forces of the tightly wound coil spring 52 and the wire 53 moving in the radial direction are smaller than those of the first to fifth embodiments in which the tightly wound coil spring 52 and the wire 53 are arranged in the vicinity of the inner peripheral surface of the flexible portion 16c, and thus anisotropy which occurs in the bending hardness of the flexible portion 16c can be suppressed.

In addition, in the sixth embodiment, the tip portions of the tightly wound coil spring 52 and the wire 53 are arranged at the center of the bending piece 40 in the radial direction via the plate spring 55. However, the present invention is not limited thereto, and as in the second embodiment, the connecting coil spring 71 may be used, or as in the third to fifth embodiments, one end of the relay member may be slidably attached to the base end piece 40B while the rotation thereof is restricted, and the tip portions of the tightly wound coil spring 52 and the wire 53 may be disposed at the center of the bending piece 40 in the radial direction.

In addition, in each of the embodiments, the tip portions of the tightly wound coil spring 52 and the wire 53 are attached to the bending piece 40 via the relay member. However, the present invention is not limited thereto, and the tip portions thereof may be attached to the connecting ring 50 positioned at the tip of the flexible portion 16c. In addition, in each of the embodiments, the plate spring 55 or the connecting coil spring 71 is used as the relay member. However, the present invention is not limited thereto, and a configuration in which the plate spring or the connecting coil spring is included in at least a portion of the relay member may be employed.

In each of the embodiments, the electronic endoscope which captures an image of the state of the inside of the body of a subject by using the camera unit is exemplified. However, the present invention is not limited thereto, and can also be applied to an endoscope which observes the state of the inside of the body of a subject by employing an optical image guide.

While the exemplary embodiments of the present invention have been described above, the present invention is not limited to the specific embodiments described above, and various changes and modifications can be made without departing from the concept of the present invention described in the appended claims.

## Claims

1. An endoscope (10) comprising:
an insertion unit which includes a tip portion (16a), a bending portion (16b), and a flexible portion (16c) arranged in order from a tip of the insertion unit (16);
a hardness varying member (52) which is disposed in the flexible portion (16c) and has a hardness that increases in accordance with compression in a tube center direction;
a hardness varying wire (53) which is inserted into the hardness varying member (52) and of which one end is fixed to one end of the hardness varying member (52), the hardness varying wire (53) configured to compress the hardness varying member (52) by a pulling operation, **characterized in that**
a relay member (55, 71) which is provided between one end of the hardness varying member (52) and a connecting ring (50) provided at a tip of the flexible portion (16c) or a bending piece (40), is more flexible than the hardness varying member (52), and is attached in a state of having deflection when the flexible portion (16c) is in a straight state;
a sliding portion (81) which is provided in one end of the relay member (55, 71) is slidably attached to the connecting ring (50) or the bending piece (40); and
a rotation restricting portion (82a, 84a, 86b) configured to restrict rotation of the sliding portion (81) with respect to the connecting ring (50) or the bending piece (40).

2. The endoscope according to claim 1, wherein
the relay member (55, 71) is made of an elastic material.

3. The endoscope according to claim 1 or 2,
wherein the sliding portion (81) includes a projection portion which protrudes in a radial direction of the hardness varying member (52), and
the rotation restricting portion is constituted by the projection portion and a groove into which the projection portion is slidably fitted.

4. The endoscope according to claim 1 or 2,
wherein the sliding portion (81) has a polygonal cross-section, and
the rotation restricting portion is constituted by the sliding portion (81) and a hole into which the sliding portion is slidably fitted and which has a polygonal cross-section.

5. The endoscope according to claim 1 or 2,
wherein the sliding portion (81) has an elliptical cross-section, and
the rotation restricting portion is constituted by the sliding portion (81) and a hole into which the sliding portion (81) is slidably fitted and which has an elliptical cross-section.

6. The endoscope according to any one of the preceding claims,
wherein the sliding portion (81) is a cylindrical member which is provided in one end of the hardness varying member (52) and into which a columnar member provided in the connecting ring or the bending piece is slidably fitted, and
the rotation restricting portion is formed in an outer peripheral surface of the columnar member and an inner peripheral surface of the sliding portion (81).

7. The endoscope according to any one of the preceding claims,
wherein the sliding portion (81) is slidably attached to a plurality of bending pieces (40).

8. The endoscope according to any one of the preceding claims,
wherein at least a portion of the relay member includes a plate spring (55) or a coil spring (52).

## Patentansprüche

1. Endoskop (10), umfassend:
eine Einführungseinheit, welche einen Spitzbereich (16a), einen Biegebereich (16b) und einen flexiblen Bereich (16c) in Reihenfolge von der Spitze der Einführungseinheit (16) umfasst;
ein Härtevariationsbauteil (52), welches in dem flexiblen Bereich (16c) angeordnet ist und eine Härte aufweist, welche mit einer Kompression in Richtung des Schlauchzentrums zunimmt;
eine Härtevariationsleitung (53), welche in das Härtevariationsbauteil (52) eingeführt ist, und
von der ein Ende an einem Ende des Härtevariationsbauteils (52) fixiert ist, wobei die Härtevariationsleitung (53) zum Kompressieren des Härtevariationsbauteils (52) mittels einer Ziehoperation eingerichtet ist, **dadurch gekennzeichnet, dass**
ein Relaisbauteil (55, 71), welches zwischen einem Ende des Härtevariationsbauteils (52) und einem Verbindungsring (50) bereitgestellt ist, wobei der Verbindungsring (50) an der Spitze des flexiblen Bereichs (16c) oder des Biegungsbereichs (40) bereitgestellt ist, flexibler ist als das Härtevariationsbauteil (52) und in einem Zustand der Deflektion, wenn der flexible Bereich (16c) in einer geraden Position ist, angefügt ist;
einen Schiebebereich (81), der an einem Ende des Relaisbauteils (55, 71) angeordnet ist und schiebbar bezüglich des Verbindungsrings (50) oder des Biegungsteils (40) angebracht ist; und
einen Rotationsbegrenzungsbereich (82a, 84a, 86b) eingerichtet zum Begrenzen der Rotation des Schiebebereichs (81) in Bezug auf den Verbindungsring (50) oder den Biegungsteil (40).

2. Endoskop gemäß Anspruch 1, wobei das Relaisbauteil (55, 71) aus elastischem Material gemacht ist.

3. Endoskop gemäß Ansprüchen 1 oder 2, wobei der Schiebebereich (81) einen Projektionsbereich, welcher radial aus dem Härtevariationsbauteil (52) heraussteht, aufweist, und
der Rotationsbegrenzungsbereich mittels des Projektionsbereichs und einer Rille, in welche der Projektionsbereich schiebbar eingepasst ist, bereitgestellt wird.

4. Endoskop gemäß Anspruch 1 oder 2, wobei der Schiebebereich (81) einen polygonalen Querschnitt aufweist, und der Rotationsbegrenzungsbereich bereitgestellt wird durch den Schiebebereich (81) und einem Loch, in welches der Schiebebereich schiebbar eingefügt ist, und der einen polygonalen Querschnitt aufweist.

5. Endoskop gemäß Anspruch 1 oder 2, wobei der Schiebebereich (81) einen elliptischen Querschnitt aufweist, und der Rotationsbegrenzungsbereich bereitgestellt wird mittels des Schiebebereichs (21) und einem Loch, in welches der Schiebebereich (81) schiebbar eingepasst ist, und welcher einen elliptischen Querschnitt aufweist.

6. Endoskop gemäß einem der vorgenannten Ansprüche, wobei der Schiebebereich (81) ein zylindrisches Bauteil ist, welches an einem Ende des Härtevariationsbauteils (52) bereitgestellt ist, und in welches ein säulenartiges Bauteil in dem Verbindungsring oder dem Biegeteil schiebbar eingepasst ist, und
der Rotationsbereich in einer äußeren peripheren Oberfläche des säulenartigen Bauteils und einer inneren peripheralen Oberfläche des Schiebebereichs (81) ausgeformt ist.

7. Endoskop gemäß einem der vorstehenden Ansprüche, wobei der Schiebebereich (21) schiebbar an einer Vielzahl von Biegungsteilen (40) angebracht ist.

8. Endoskop gemäß einem der vorgenannten Ansprüche, wobei zumindest ein Teil des Relaisbauteils eine Tellerfeder (55) oder eine Spiraldruckfeder (52) aufweist.

## Revendications

1. Endoscope (10), comprenant :
une unité d'introduction, laquelle inclut une portion de bout (16a), une portion de flexion (16b) et une portion flexible (16c) agencées dans cet ordre à partir d'un bout de l'unité d'introduction (16) ;
un élément variant en dureté (52), disposé dans la portion flexible (16c) et présentant une dureté qui augmente en fonction de la compression dans une direction centrale de tube ;
un fil variant en dureté (53), introduit dans l'élément variant en dureté (52), et dont une extrémité est fixée à une extrémité de l'élément variant en dureté (52), le fil variant en dureté (53) étant configuré pour comprimer l'élément variant en dureté (52) par une opération de traction, **caractérisé en ce que**
un élément relais (55, 71), prévu entre une extrémité de l'élément variant en dureté (52) et un anneau de raccordement (50) prévu sur un bout de la portion flexible (16c) ou une pièce de flexion (40), est plus flexible que l'élément variant en dureté (52), et est attaché dans un état présentant une déviation lorsque la portion flexible (16c) se trouve dans un état droit ;
une portion coulissante (81), prévue sur une extrémité de l'élément relais (55, 71) est attachée d'une manière permettant le coulissement sur l'anneau de raccordement (50) ou la pièce de flexion (40), et
une portion de restriction de rotation (82a, 84a, 86b) configurée pour restreindre la rotation de la portion coulissante (81) par rapport à l'anneau de raccordement (50) ou la pièce de flexion (40).

2. Endoscope selon la revendication 1, dans lequel
l'élément relais (55, 71) est fabriqué dans un matériau élastique.

3. Endoscope selon la revendication 1 ou 2,
dans lequel la portion coulissante (81) inclut une portion de saillie, laquelle fait saillie dans une direction radiale de l'élément variant en dureté (52), et
la portion de restriction de rotation est constituée de la portion de saillie et d'une rainure dans laquelle la portion de saillie est montée d'une manière permettant le coulissement.

4. Endoscope selon la revendication 1 ou 2,
dans lequel la portion coulissante (81) présente une section polygonale, et
la portion de restriction de rotation est constituée par la portion coulissante (81) et un trou dans lequel la portion coulissante est montée d'une manière permettant le coulissement et qui présente une section polygonale.

5. Endoscope selon la revendication 1 ou 2,
dans lequel la portion coulissante (81) présente une section elliptique, et
la portion de restriction de rotation est constituée par la portion coulissante (81) et un trou dans lequel la portion coulissante (81) est montée d'une manière permettant le coulissement et qui présente une section elliptique.

6. Endoscope selon l'une quelconque des revendications précédentes,
dans lequel la portion coulissante (81) est un élément cylindrique prévu dans une extrémité de l'élément variant en dureté (52) et dans lequel un élément en colonnes prévu dans l'anneau de raccordement ou la pièce de flexion est monté d'une manière permettant le coulissement, et
la portion de restriction de rotation est formée dans une surface périphérique extérieure de l'élément en colonnes et une surface périphérique intérieure de la portion coulissante (81).

7. Endoscope selon l'une quelconque des revendications précédentes,
dans lequel la portion coulissante (81) est attachée d'une manière permettant le coulissement sur une pluralité de pièces de flexion (40).

8. Endoscope selon l'une quelconque des revendications précédentes,
dans lequel au moins une portion de l'élément de relais inclut un ressort à lames (55) ou un ressort à enroulement (52).
